# FASCICULE DE BREVET EUROPEEN

(11) **EP 2 572 782 B1**
(45) Date de publication et mention de la délivrance du brevet: **22.10.2014**
(21) Numéro de dépôt: 12290287.7
(22) Date de dépôt: 31.08.2012
(51) Int. Cl.: C07C 7/177, C07C 11/08

(54) **Procédé de séparation du butène-2 d'une coupe C4 contenant du butène-2 et du butène-1 par oligomérisation sélective du butène-1**
Separationsverfahren von Buten-2 eines C4-Verschnitts, der Buten-2 und Buten-1 enthält, durch selektive Oligomerisation von Buten-1
Method for separating butene-2 from a C4 cut containing butene-2 and butene-1 by selective oligomerisation of the butene-1

(30) Priorité: 20.09.2011 FR 1102849
(43) Date de publication de la demande: 27.03.2013
(73) Titulaire: IFP Energies nouvelles, 92852 Rueil-Malmaison Cedex (FR)
(72) Inventeur: Breuil, Pierre-Alain, 69006 Lyon (FR); Olivier-Bourbigou, Helene, 69230 Saint Genis-Laval (FR)

(56) Documents cités:
- EP-A1- 0 079 679
- US-A- 2 375 687
- US-A- 2 826 620
- US-A1- 2002 177 744

## Description

La présente invention décrit un procédé de séparation du butène-2 d'une coupe C4 contenant du butène-2 et du butène-1 par oligomérisation sélective du butène-1 en oligomères majoritairement linéaires en présence d'une composition catalytique à base de fer. Des étapes optionnelles supplémentaires permettent de séparer l'isobutène, le butadiène-1,3 ainsi que des traces d'hydrocarbures acétyléniques optionnellement présents dans la charge C4.

Les coupes C4 sont disponibles en grande quantité dans les raffineries et les sites pétrochimiques. Elles sont produites par les procédés classiques tels que le craquage catalytique ou le craquage à la vapeur. Les coupes C4 peuvent également être issues de la biomasse après la déshydratation des alcools. Ces coupes C4 contiennent typiquement des composés oléfiniques tels que des n-butènes (butène-1 et butènes-2), de l'isobutène et optionnellement des paraffines (isobutane et n-butane), des diènes (tel que le butadiène-1,3) ainsi que des impuretés acétyléniques. Le tableau 1 montre les compositions types de coupes C4 issues d'un craquage catalytique à lit fluidisé, d'un craquage à la vapeur et d'une déshydratation d'alcools.

**Tableau 1 : Compositions types coupes C4**

| | Composition typique (%pds) craquage catalytique à lit fluidisé * | Composition (%pds) Craquage à la vapeur * | Composition (%pds) Déshydratation des alcools |
|---|---|---|---|
| n-Butane | 11 | 5 | - |
| Butène-1 | 13 | 28 | 20 |
| Butène-2 | 26 | 20 | 20 |
| Isobutène | 15-25 | 45-50 | 40 |
| Isobutane | 35 | 2 | - |
| Butadiene | 0.5 | 1 | - |

| | | | |
|---|---|---|---|
| * après hydrogénation sélective | | | |

Quel que soit le procédé, leur séparation reste souvent problématique. Compte tenu de la faible différence des points d'ébullition de ces composés (voir Tableau 2), il est difficile de les séparer par distillation, en particulier la séparation par distillation du butène-1 et de l'isobutène n'est pas réalisable. C'est pourquoi des techniques de séparation physique et chimique plus efficaces et sélectives doivent être mises en oeuvre.

**Tableau 2 : Point ébullition des composés C4 :**

| | Point d'ebullition (°C) |
|---|---|
| n-Butane | -0,4 |
| Butène-1 | -6,3 |
| Butène-2 Cis | 3,7 |
| Butène-2 Trans | 0,9 |
| Isobutène | -6,9 |
| Isobutane | -11,7 |

Industriellement, la séparation de la coupe C4 commence généralement par la séparation du butadiène, par exemple par extraction liquide-liquide avec un solvant (N-méthyl-2-pyrrolidone, DMF, acétone, acétonitrile...) et/ou par hydrogénation sélective permettant d'obtenir un mélange d'isobutènes, de n-butènes et de butanes. L'isobutène est ensuite séparé des autres composées C4 puisqu'il est ramifié et qu'il présente une réactivité différente. La séparation de l'isobutène est réalisée par des voies détournées en le transformant par des procédés tels que l'éthérification par le méthanol ou l'oligomérisation sélective ou la polymérisation conduisant respectivement le plus typiquement au MTBE (méthyl-tert-butyl-éther), au düsobutène ou PIB (polyisobutène). Ce sont les débouchés qui orientent ses voies. L'isobutène pur peut être régénéré à partir du MTBE par craquage. Le brevet EP0869107 décrit ainsi un procédé de production d'alpha oléfines (butène-1), d'oléfine tertiaire (isobutène) et/ou d'éther à partir de coupe hydrocarbonée insaturée.
Après élimination de l'isobutène, il ne reste que les n-butènes et les butanes. On ne procède généralement pas à une nouvelle séparation, puisque les alcanes ne réagissent pas au cours des réactions de transformation des butènes et sont donc extraits comme des inertes. En principe, la séparation par distillation du butène-2 (cis et trans) du butène-1 est possible mais reste difficile. Les butanes peuvent être séparés des n-butènes par distillation extractive.
D'autres développements permettent des séparations plus poussées de la coupe C4, et notamment une séparation des n-butènes. Ces procédés nécessitent souvent plusieurs étapes. Ainsi, le brevet EP0079679 décrit un procédé de séparation de butène-1 du butène-2 par deux étapes de distillation plus deux étapes de distillation extractive. Le brevet US 2 826 620 décrit un procédé de séparation d'une oléfine alpha d'un mélange d'hydrocarbures mettant en oeuvre une réaction de polymérisation de l'oléfine alpha avec un catalyseur à base d'oxyde de chrome. Tous les butènes sont des matières premières intéressantes en synthèse industrielle. Le butène-2 contenu dans les coupes C4 peut être utilisé comme substrat pour la production de triméthylpentènes (isooctanes), utilisés comme additifs à haut indice d'octane pour carburant, par un procédé d'alkylation avec l'isobutane.
Il peut également être utilisé en combinaison avec l'éthylène pour la production de propylène en mettant en oeuvre une réaction de métathèse croisée. Par exemple, le brevet US5898091 décrit la production conjointe de propylène et d'éther à partir d'une coupe C4 par un procédé mettant en oeuvre les différentes étapes suivantes : 1) hydroisomérisation des diènes, 2) éthérification isobutène en MTBE, 3) séparation des composés oxygénés, 4) métathèse du butène-2 avec de l'éthylène. Le brevet US 6207115 décrit la production conjointe de propylène et de polyisobutène par un procédé mettant en oeuvre les étapes suivantes : 1) hydroisomérisation des diènes 2) polymérisation de l'isobutène 3) métathèse du butène-2 avec l'éthylène.
Les butènes linéaires issus de la coupe C4 peuvent également être utilisés pour la production d'octènes majoritairement ramifiés par un procédé d'oligomérisation (Dimersol) ou pour la production de *sec*-butanol et de methyléthylcétone (MEK).

Il existe d'un autre côté des procédés l'oligomérisation d'oléfines alpha linéaires. Le brevet US2002/0177744 décrit la synthèse de dimères du butène-1 et d'autres oléfines alpha linéaires par couplage d'alpha-oléfines catalysée par des complexes du fer et du cobalt activés par un dérivé de l'aluminium. Les dimères en C8 formés (octènes) ont la particularité d'avoir une très bonne linéarité.
Les octènes majoritairement linéaires peuvent être utilisés avantageusement pour
la synthèse d'aldéhydes ou d'alcools après hydroformylation et hydrogénation. Ces alcools peuvent être utilisés par exemple pour la production de phtalates (plastifiants) ayant des propriétés améliorées.
Il a maintenant été trouvé que ces complexes du fer pouvaient être utilisés pour transformer de façon sélective le butène-1 par une réaction d'oligomérisation lorsque celui-ci est en mélange avec une charge contenant du butène-2 et optionnellement de l'isobutane, du n-butane et/ou de l'isobutène.

L'oligomérisation des oléfines légères (C2-C4) par les complexes de métaux de transition est majoritairement décrite dans la littérature dans le cas de l'éthylène. Il est en effet bien connu que la réactivité des oléfines décroit lorsque la longueur de leur chaîne carbonée croit (C2>>C3>C4). Les systèmes les plus connus pour transformer les butènes par oligomérisation sont les systèmes de type Ziegler à base de nickel. Ces systèmes transforment le butène-1 et le butène-2 en un mélange d'octènes (pour référence voir *"*Reaction of unsaturated compounds", p240-253 dans "Applied Homogeneous Catalysis with Organometallics" Ed. B. Cornils, W. Herrmann, Wiley-VCH, 2002).

Les systèmes à base de fer selon l'invention se différencient donc des systèmes connus de la littérature. Il est en effet surprenant que le système catalytique ne réagisse pas ou très peu avec le butène-2.

La présente invention a donc pour objectif de proposer un procédé de séparation du butène-2 d'une charge C4 comprenant du butène-1 et du butène-2 caractérisé par les étapes successives suivantes:
- une étape d'oligomérisation sélective du butène-1 par une composition catalytique à base de fer
- une étape de séparation des oligomères formés du butène-2.

Le procédé selon l'invention permet ainsi d'une part de séparer efficacement le butène-2 du butène-1 et d'autre part de produire en même temps des octènes majoritairement linéaires utilisables par exemple avantageusement comme matière première pour l'hydroformylation pour produire des précurseurs pour la production de plastifiants pour le polychlorure de vinyle (PVC). Le procédé de séparation selon l'invention possède notamment l'avantage d'être un procédé simple pouvant être effectué avec moins d'étapes que les procédés de l'état de l'art tel que celui décrit dans EP0079679.

D'autres étapes de séparation peuvent s'ajouter à l'étape d'oligomérisation dans le cas d'une charge contenant en plus du butène-1 et du butène 2 d'autres composants C4 tels que l'isobutène et/ou le butadiène-1,3 et/ou des impuretés acétyléniques. Dans le cas où la charge contient en plus de l'isobutène, on procède de préférence à une étape de séparation de l'isobutène avant l'étape d'oligomérisation sélective. Dans le cas où la charge contient du butadiène-1,3 et des traces d'hydrocarbures acétyléniques, on procède de préférence à une étape de séparation du butadiène-1,3 et des traces d'hydrocarbures acétyléniques par hydrogénation sélective avant l'étape de séparation de l'isobutène (optionelle) et/ou avant l'étape d'oligomérisation sélective.

Ainsi, selon une variante, le procédé de séparation du butène-2 d'une charge C4 contenant du butène-1, du butène-2, de l'isobutène, du butadiène-1,3 et des impuretés acétyléniques, comprend avantageusement la suite des étapes successives suivantes :
- une étape optionnelle d'hydrogénation sélective du butadiène-1,3 et des impuretés acétyléniques,
- une étape optionnelle de séparation de l'isobutène par éthérification, dimérisation ou polymérisation,
- une étape d'oligomérisation sélective du butène-1 conduisant à la séparation du butène-2 et à la co-production d'oligomères majoritairement linéaires.
La succession de ces étapes présente de nombreux avantages. Les composés les plus réactifs de la coupe, à savoir le butadiène-1,3 et les traces d'hydrocarbures acétyléniques, sont transformés dès la première étape, et ne seront donc pas la cause de réactions parasites dans les étapes suivantes. Par ailleurs l'hydroisomérisation sélective du butadiène-1,3 permet d'augmenter la concentration en butène-2, ce qui favorise le rendement de ce produit recherché.
La séparation optionnelle de l'isobutène permet d'obtenir de l'isobutène utilisable pour la production de polyisobutène, de diisobutène ou d'isobutène haute pureté.

### Description détaillée

### La charge

La charge mise en oeuvre est une coupe d'hydrocarbures C4 contenant du butène-1 et du butène-2. Elle peut en plus contenir de l'isobutène et/ou du butadiène-1,3 et/ ou des impuretés acétyléniques. Typiquement, elle contient des butènes (butène-1 et butène-2), de l'isobutène, des paraffines (isobutane et n-butane), du butadiène-1,3 et des traces d'hydrocarbures acétyléniques. Ces charges peuvent provenir d'une unité de craquage catalytique, par exemple en lit fluidisé, d'une unité de vapocraquage (de naphta), et/ou d'une unité de déshydratation des alcools ou d'autres sources.

Suivant les différents composants de la charge, une ou plusieurs étapes de séparation peuvent avantageusement précéder l'étape de séparation du butène-2 par oligomérisation sélective du butène-1. Il s'agit notamment d'une étape de séparation du butadiène-1,3 et des impuretés acétyléniques par hydrogénation sélective et d'une étape de séparation de i'isobutène. Puisque ces étapes précèdent l'étape d'oligomérisation, elles seront décrites par la suite, avant la description de l'étape d'oligomérisation sélective.

### Hydrogénation sélective

La charge contenant en plus du butène-1 et du butène-2 du butadiène-1,3 et des traces d'hydrocarbures acétyléniques peut être soumise à une étape d'hydrogénation sélective.

L'objet principal de cette première étape optionnelle est de transformer le butadiène-1,3 en butènes. Le deuxième objet de cette étape est d'éliminer les traces d'hydrocarbures acétyléniques toujours présentes dans ces coupes et qui sont des poisons ou des polluants pour les étapes ultérieures. En effet, cette étape permet de transformer les composés les plus réactifs de la coupe, à savoir le butadiène-1,3 et les composés acétyléniques dès la première étape, et ne seront donc pas la cause de réactions parasites dans les étapes suivantes. De même, cette réaction permet d'augmenter le rendement en n-butènes.
Une autre réaction intervenant dans l'hydrogénation sélective est l'isomérisation du butène-1 en butène-2 à l'équilibre thermodynamique.

Dans cette première étape optionnelle d'hydrogénation sélective, on réalise donc simultanément les réactions suivantes:
- hydroisomérisation sélective du butadiène-1,3 en un mélange de n-butènes à l'équilibre thermodynamique,
- isomérisation du butène-1 en butène-2, également à l'équilibre thermodynamique,
- hydrogénation des traces d'hydrocarbures acétyléniques.

Dans le cas d'une charge venant d'un vapocraqueur, on procède de préférence à une étape d'hydrogénation sélective des diènes. Dans le cas d'une charge venant d'une unité de craquage catalytique, par exemple en lit fluidisé, l'étape d'hydrogénation sélective des diènes est optionnelle. Si la concentration en dioléfines ou acétylène dans la coupe utilisée pour ledit procédé est supérieure à 1000 ppm, l'hydrogénation sélective est recommandée afin de réduire cette concentration en dessous de 1000 ppm. Les charges contenant plus de 300 ppm de ces impuretés ou même plus de 10 ppm seront de manière préférée traitée par cette étape.

Les réactions peuvent être réalisées au moyen de divers catalyseurs spécifiques comprenant un ou plusieurs métaux, par exemple du groupe 10 de la classification périodique (Ni, Pd, Pt), déposés sur un support. On met en oeuvre de préférence un catalyseur qui comprend au moins un composé de palladium fixé sur un support minéral réfractaire, par exemple sur une alumine. La teneur en palladium sur le support peut être comprise entre 0,01 et 5 % en poids, de préférence entre 0,05 et 1 % en poids. Divers modes de prétraitement connus de l'homme de l'art peuvent éventuellement être appliqués à ces catalyseurs pour améliorer la sélectivité dans l'hydrogénation du butadiène-1,3 en butènes aux dépens de l'hydrogénation totale en butane qu'il faut éviter. Le catalyseur contient de préférence 0,05 à 10 % en poids de soufre. De façon avantageuse, on utilise un catalyseur constitué par du palladium déposé sur de l'alumine et qui contient du soufre.

La sulfuration du catalyseur peut être effectuée in situ (dans la zone de réaction) ou mieux ex situ. Dans ce dernier cas, le catalyseur a été avantageusement traité, avant son chargement dans le réacteur d'hydrogénation, par au moins un composé soufré dilué dans un solvant. Le catalyseur obtenu contenant 0,05 à 10 % de soufre (en poids) est chargé dans le réacteur et activé sous atmosphère neutre ou réductrice à une température comprise entre 20 et 300°C, une pression comprise entre 0,1 et 5 MPa et une VVH comprise entre 50 et 600 h⁻¹, et la charge est mise au contact dudit catalyseur activé.

La mise en oeuvre du catalyseur, de préférence au palladium, n'est pas critique, mais on préfère généralement utiliser au moins un réacteur à flux descendant à travers un lit fixe de catalyseur. Lorsque la proportion de butadiène-1,3 dans la coupe est importante, ce qui est le cas par exemple d'une coupe de vapocraquage lorsqu'on ne souhaite pas en extraire le butadiène-1,3 pour des usages spécifiques, il peut être avantageux d'effectuer la transformation dans deux réacteurs en série afin de mieux contrôler la sélectivité de l'hydrogénation. Le second réacteur peut être à flux ascendant et avoir un rôle de finition.

La quantité d'hydrogène nécessaire à l'ensemble des réactions réalisées dans cette étape est ajustée en fonction de la composition de la coupe pour avoir avantageusement seulement un léger excès d'hydrogène par rapport à la stoechiométrie théorique.

Les conditions opératoires sont choisies de telle façon que les réactifs et les produits soient en phase liquide. Il peut cependant être avantageux de choisir un mode de fonctionnement tel que les produits soient partiellement vaporisés à la sortie du réacteur, ce qui facilite le contrôle thermique de la réaction. La température peut varier de 20 à 200°C, de préférence de 50 à 150°C ou mieux de 60 à 150°C. La pression peut être ajustée entre 0,1 et 5 MPa, de préférence entre 0,5 et 4 MPa et avantageusement entre 0,5 et 3 MPa, de telle façon que les réactifs, au moins en partie, soient en phase liquide. La vitesse spatiale peut être comprise entre 0,5 et 10 h⁻¹ et de préférence entre 1 et 6 h⁻¹, avec un rapport H2/dioléfines (molaire) de 0,5 à 5 et de préférence de 1 à 3.

De préférence, l'hydrogénation sélective du butadiène-1,3 et des traces d'hydrocarbures acétyléniques est effectuée par un catalyseur comprenant au moins un métal choisi dans le groupe formé par le nickel, le palladium et le platine, déposé sur un support, à une température de 20-200°C, une pression de 1-5 MPa, une vitesse spatiale de 0,5-10 h⁻¹ et avec un rapport H2 / butadiène-1, 3 (molaire) de 0,5 à 5.

Le ou les réacteurs d'hydroisomérisation peuvent avantageusement être suivis d'une colonne de stabilisation qui élimine les traces d'hydrogène en excès et le méthane éventuel.

### Séparation de l'isobutène

La charge contenant en plus du butène-1 et du butène-2 de l'isobutène peut être soumise à une étape de séparation de l'isobutène. Cette étape optionnelle intervient de préférence après l'étape optionnelle d'hydrogénation sélective mais avant l'étape d'oligomérisation.
D'une manière générale, la séparation de l'isobutène peut être effectuée par au moins une des étapes suivantes :
- éthérification de l'isobutène en un alkyl-tertiobutyléther en présence d'un catalyseur d'étherification avec un alcool dont la chaîne hydrocarbonée peut comporter de 1 à 10 atomes de carbone,
- dimérisation de l'isobutène en présence d'un catalyseur acide,
- polymérisation de l'isobutène en présence d'un catalyseur acide de Lewis. conduisant respectivement le plus typiquement au MTBE (méthyl-tert-butyl-éther), au diisobutène ou PIB (polyisobutène).
De préférence, la séparation de l'isobutène est effectuée par éthérification.

### Éthérification

La charge contenant du butène-1, du butène-2 et de l'isobutène, et de préférence débarrassée du butadiène-1,3 et des traces d'hydrocarbures acétyléniques, peut être soumise à une éthérification afin de former un éther alkylique tertiaire par mise en contact dans une zone réactionnelle contenant un catalyseur d'éthérification. L'étape d'éthérification est connue par l'homme du métier.

L'objet de la deuxième étape optionnelle est de transformer l'isobutène présent dans la coupe C4 en un alkyl-tertiobutyléther par éthérification avec un alcool dont la chaîne hydrocarbonée peut comporter de 1 à 10 atomes de carbone. On utilise de préférence comme alcool le méthanol ou l'éthanol. Les éthers produits sont respectivement le méthyl-tert-butyléther (MTBE) et l'éthyl-tert-butyléther (ETBE).

La transformation est réalisée au moyen d'un catalyseur acide, par exemple un catalyseur à base d'une résine échangeuse d'ions sous forme H⁺, comme une résine sulfonique -SO₃H. Ce catalyseur peut être mis en oeuvre par exemple en lit fixe de façon conventionnelle, ou en lit mobile. On préfère opérer avec un lit expansé de catalyseur, entretenu par une circulation à flux ascendant du milieu réactionnel à travers le réacteur et un échangeur de chaleur extérieur. Cette façon d'opérer permet de contrôler aisément le fonctionnement du catalyseur ainsi que l'élimination des calories produites dans la réaction, en évitant la formation de points chauds.

Un réacteur de finition peut avantageusement être placé à la suite du réacteur à lit expansé pour épuiser au maximum l'isobutène dans la coupe résiduelle et augmenter le rendement en éther, mais la majeure partie de la réaction se produit dans le réacteur à lit expansé. On peut également épuiser au maximum l'isobutène en mettant en oeuvre, en finition, une distillation réactive (colonne de distillation contenant du catalyseur) qui permet d'augmenter la conversion grâce à la séparation des produits au niveau du réacteur.
Les conditions opératoires sont choisies de telle façon que les réactifs et les produits soient en phase liquide. On fixe en général la température pour que la réaction se déroule à une vitesse suffisante, par exemple de 30 à 130°C, de préférence de 40 à 100°C, et la pression est ajustée en conséquence de telle façon que les réactifs soient en phase liquide.

Un fractionnement du produit issu de l'étape d'éthérification permet d'obtenir d'une part une fraction organique enrichie en éther alkylique tertiaire et d'autre part une fraction organique appauvrie en éther alkylique tertiaire contenant le butène-1 et le butène-2 et éventuellement les butanes, si présents. Ainsi, la section réactionnelle est suivie d'une section de distillation où on sépare l'éther en fond de colonne et un distillat comprenant la coupe C4 résiduelle et l'alcool en excès ainsi que des traces d'autres composés oxygénés volatils. L'alcool est séparé par un lavage à l'eau et le mélange eau-alcool est distillé pour recycler l'alcool.

Les éthers ainsi produit tels que le MTBE ou l'ETBE peuvent être utilisés comme additifs pour augmenter l'indice d'octane dans les essences reformulées. Il est également possible de récupérer l'isobutène à partir des éthers produit qui sont facilement décomposés en présence d'un catalyseur acide pour libérer l'isobutène purifié.

### Dimérisation de l'isobutène

La charge contenant du butène-1, du butène -2 et de l'isobutène, et de préférence débarrassée du butadiène-1,3 et des traces d'hydrocarbures acétyléniques, peut également être soumise à une dimérisation de l'isobutène en "di-isobutène", un mélange d'isomères contenant principalement le 2, 2, 4- triméthyl-pentène (isooctane). L'étape de dimérisation de l'isobutène est connue par l'homme du métier.

Cette réaction s'effectue en présence d'un catalyseur acide tel par exemple l'acide sulfurique ou des résines échangeuses d'ions acides, à des températures entre 20 et 130°C, de préférence entre 60 et 130°C et de manière encore plus préférée entre 90 et 110°C.

Pour réaliser cette réaction, on peut également utiliser comme catalyseur et comme solvant une composition comprenant au moins un acide de Brønsted, noté HB, dissous dans un milieu liquide ionique comprenant au moins un cation organique Q⁺ et un anion A⁻, comme cela est décrit dans le brevet US7256152.

### Polymérisation de l'isobutène

La charge contenant du butène-1, du butène -2 et de l'isobutène, et de préférence débarrassée du butadiène-1,3 et des traces d'hydrocarbures acétyléniques, peut également être soumise à une polymérisation de l'isobutène en polyisobutène (PIB) pouvant entrer dans la composition d'élastomères, ou encore en polybutènes liquides utilisables comme additifs de lubrifiants. L'étape de polymérisation de l'isobutène est connue par l'homme du métier.

La polymérisation est réalisée au moyen d'un catalyseur acide de Lewis, par exemple du chlorure d'aluminium, un chloroalkylaluminium, du tétrachlorure d'étain, du trifluorure de bore, éventuellement associé à des traces d'acides de Bronsted comme l'acide chlorhydrique, l'eau, le chlorure de butyle tertiaire, les acides organiques. Le catalyseur peut être mis en oeuvre à l'état solide en poudre ou sous forme d'une suspension dans un hydrocarbure saturé tel que l'hexane ou l'isobutane, ou dans un hydrocarbure halogéné tel que le chlorure de méthyle.

Les conditions opératoires sont choisies de telle façon que la température de réaction puisse être contrôlée avec précision. On fixe en général la température pour que la réaction se déroule à une vitesse suffisante, par exemple de -100 à +100°C, de préférence de -50 à +50°C, et la pression est ajustée par exemple de telle façon que l'hydrocarbure éventuellement mis en oeuvre soit partiellement vaporisé par la chaleur dégagée par la polymérisation. Dans le cas ou le catalyseur est mis en oeuvre à l'état solide, la chaleur de réaction peut être enlevée par la circulation en boucle du mélange réactionnel à travers un échangeur extérieur.

La section réactionnelle est suivie d'une section dans laquelle le catalyseur est séparé de l'effluent, par exemple par un lavage avec de la soude suivi d'un lavage à l'eau, puis d'une section de séparation (par exemple de distillation) où on sépare le polymère.
Il peut être intéressant, pour améliorer la qualité des produits, d'extraire l'isobutène de l'effluent résultant de l'étape précédente avant polymérisation. Cette extraction peut se faire par toute méthode bien connue, par exemple simplement par distillation, ou bien par hydratation de l'isobutène en alcool butylique tertiaire en présence par exemple d'acide sulfurique, suivie d'une séparation et d'une déshydratation de l'alcool pour récupérer l'isobutène, ou bien encore par réaction avec du méthanol en présence d'une résine acide échangeuse d'ions pour transformer l'isobutène en méthyl-tert-butyléther (MTBE) qui est facilement séparé par distillation puis décomposé en présence d'un catalyseur acide pour libérer l'isobutène purifié.

La coupe C4 résiduelle ainsi obtenue contient du butène-2, du butène-1 ainsi qu'éventuellement des butanes. Elle est soumise à l'étape d'oligomérisation sélective.

### Oligomérisation sélective

La charge contenant du butène-1 et du butène-2, et de préférence débarrassée du butadiène-1,3 et des traces d'hydrocarbures acétyléniques et optionnellement débarrassée de l'isobutène, est soumise à une étape d'oligomérisation sélective du butène-1 par des compositions catalytiques à base de fer sans que le butène-2 réagisse d'une façon significative.
Cette étape conduit principalement à la formation d'octènes, majoritairement linéaires. Dans le cas d'une charge contenant des C4, la sélectivité en dimères (octènes) est généralement supérieure à 50%, de préférence supérieure à 70% par rapport aux oléfines transformées. La sélectivité en dimères linéaires (n-octènes) par rapport à l'ensemble des dimères formés (octènes) est généralement supérieure à 50%, de préférence supérieure à 60%.

La composition catalytique à base der fer utilisée dans l'étape d'oligomérisation sélective est composée d'au moins un précurseur de fer, au moins un ligand de formule décrite ci-dessous, complexé ou non au précurseur de fer, et optionnellement d'un agent activateur.

Le précurseur de fer a pour formule générale FeXₙ, X étant un groupement anionique, par exemple un halogénure tel qu'un chlorure, un fluorure, un bromure ou un iodure; ou un groupement hydrocarboné, par exemple un méthyle, un benzyle ou un phényle; un carboxylate, par exemple un acétate ou un acétylacétonate; un oxyde; un amide, par exemple un diéthyle amide; un alkoxyde, par exemple un méthoxyde, un éthoxyde ou un phénoxyde; ou un hydroxyle. Alternativement, X peut être un anion non-coordinant par exemple un tétrafluoroborate, un aryle borate fluoré ou un triflate. Le groupement anionique X peut être monoanionique ou dianionique. Les précurseurs de fer peuvent être hydratés ou non, coordinés ou non avec un ligand.

A titre d'exemple, les précurseurs de fer peuvent être le chlorure de fer(II), le chlorure de fer(III), le fluorure de fer (II), le fluorure de fer(III), le bromure de fer(II), le bromure de fer(III), l'iodure de fer(II), l'iodure de fer(III), l'acétate de fer(II), l'acétate de fer(III), l'acétylacétonate de fer(II), l'acétylacétonate de fer(III), l'octoate de fer(II), l'octoate de fer(III), le 2-éthylhexanoate de fer(II), le 2-éthylhexanoate de fer(III), le triflate de fer(II), le triflate de fer(III), le nitrate de fer(III). Les précurseurs de fer peuvent être hydratés ou non, coordinés ou non avec un ligand comme le tétrahydrofurane par exemple.

Le ligand a pour formule générale : 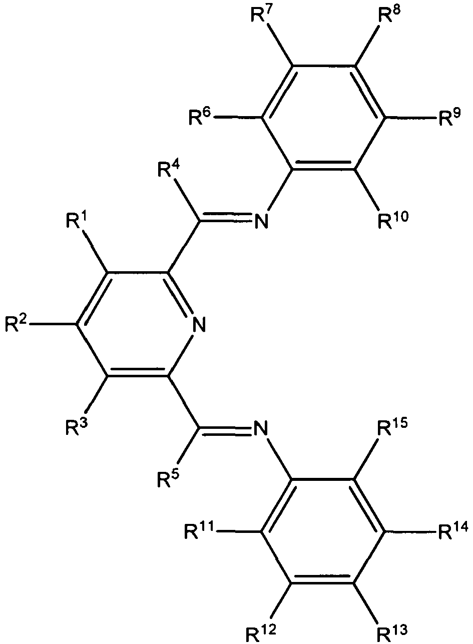 où R¹, R², R³, R⁴, R⁵, R⁶, R⁷, R⁸, R⁹, R¹⁰, R¹¹, R¹², R¹³, R¹⁴ et R¹⁵ peuvent être identiques ou différents, sont choisis parmi l'atome d'hydrogène, les groupements alkyles linéaires ou ramifiés, cycliques ou non, saturés ou insaturés, les groupements aryles, aralkyles ou alkaryles comportant 1 à 12 atomes de carbone, les groupements contenant des hétéro-éléments, hétérocycliques ou non, aromatiques ou non, halogénures ou non, supportés ou non, et de préférence choisi parmi le groupe alcoxyle, nitro, halogénures et/ou perfluoroalkyle.

A titre d'exemples non limitatifs, R¹, R², R³, R⁴, R⁵, R⁶, R⁷, R⁸, R⁹, R¹⁰, R¹¹, R¹², R¹³, R¹⁴ et R¹⁵ peuvent être choisis parmi les groupements hydrogène, méthyle, éthyle, isopropyle, *iso*-butyle, *tert*-butyle, cyclohexyle, phényle, benzyle, méthoxy, nitro, diméthylamine, diéthylamine, diisopropylamine, trifluorométhyle, fluorure, chlorure, bromure et iodure.

A titre d'exemple, les ligands peuvent être de formule ci-dessous :

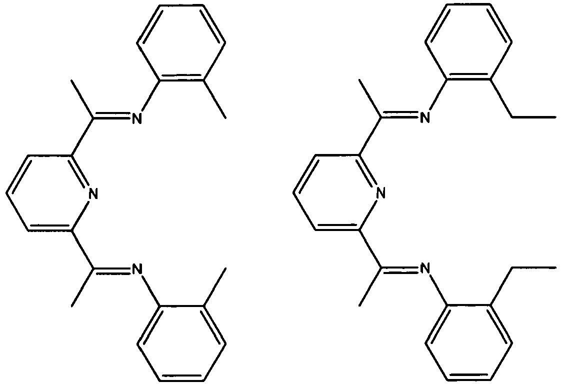

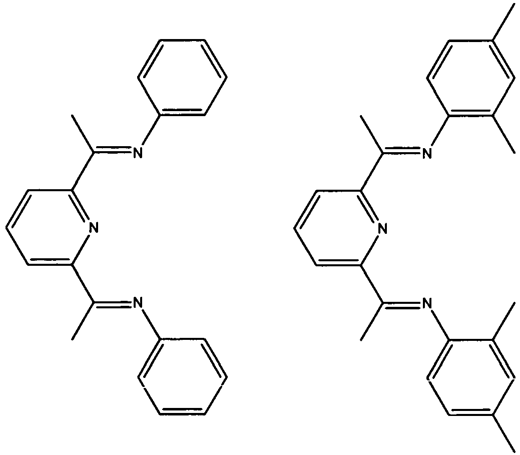

A titre d'exemple, les complexes peuvent être de formule ci-dessous :

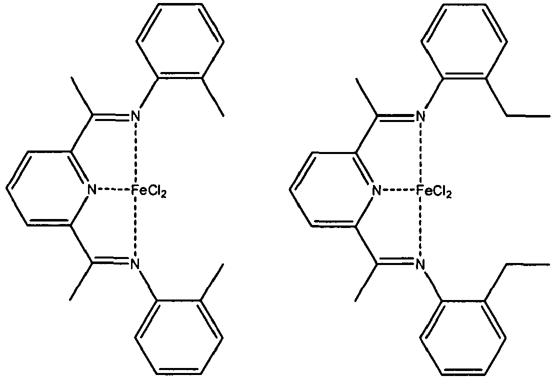

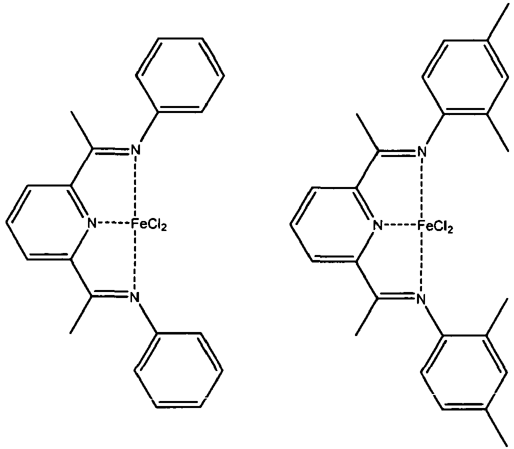

L'activateur optionnel pour le catalyseur utilisé dans la présente invention est préférentiellement un acide de Lewis. De manière préférentielle, l'acide de Lewis est choisi parmi les dérivés d'aluminium et les dérivés du bore ou du zinc ou un mélange de ces dérivés.

Comme exemples des dérivés d'aluminium peuvent être inclus les alkylaluminiums, comme par exemple le triméthylaluminium, le triéthylaluminium, le tributylaluminium, le tri-*n*-octylaluminium; les halogénures d'alkylaluminiums, comme par exemple le chlorure de diéthylaluminium, le dichlorure d'éthylaluminium, le sesquichlorure d'éthylaluminium, le dichlorure de méthylaluminium, le dichlorure d'isobutylaluminium; et les aluminoxanes. Les aluminoxanes sont bien connus par l'homme de l'art comme des composés oligomériques pouvant être préparés par l'addition contrôlée d'eau sur un alkylaluminium, par exemple le triméthylaluminium. De tels composés peuvent être linéaires, cycliques ou des mélanges de ces composés. Ils sont généralement représentés par la formule [RAIO]ₐ où R est un groupement hydrocarboné et a est un nombre de 2 à 50. Préférentiellement, l'aluminoxane est choisi parmi le méthylaluminoxane (MAO) et/ou l'éthylaluminoxane (EAO) et/ou parmi les aluminoxanes modifiés tels que le méthylaluminoxane modifié (MMAO).

Comme exemples des dérivés du bore peuvent être inclus les trialkylboranes, comme par exemple le triméthylborane, le triéthylborane, le tripropylborane, le tri-n-butylborane, le tri-isobutylborane, le tri-n-hexylborane, le tri-n-octylborane, utilisé seul ou en mélange, les tris(aryl)boranes, comme par exemple le tris(perfluorophényl)borane, le tris(3,5-bis(trifluorométhyl)phényl)borane, le tris(2,3,4,6-tétrafluorophényl)borane, le tris(perfluoronaphtyl)borane, le tris(perfluobiphényl)borane et leurs dérivés. Il est aussi possible d'utiliser comme activateur les (aryl)borates associés à un cation triphénylcarbénium ou à un cation ammonium trisubstitué tels que le triphénylcarbenium tétrakis(perfluorophényl)borate, le N,N-diméthylanilinium tétrakis(perfluorophényl)borate, le N,N-diéthylanilinium tétrakis(3,5-bis(trifluorométhyl)phényl)borate, le triphénylcarbenium tétrakis(3,5-bis(trifluorométhyl)phényl)borate.

Comme exemples des dérivés du zinc peuvent être inclus les dialkylzinc, comme par exemple le diméthylzinc, le diéthylzinc, le dipropylzinc, le dibutylzinc,le dinéopentylzinc, le di(triméthylsilyleméthyl)zinc, utilisé seul ou en mélange.

L'oligomérisation sélective du butène-1 peut être réalisée en présence d'un solvant. Le solvant organique utilisé dans la composition catalytique sera de préférence un solvant aprotique. Parmi les solvants qui peuvent être utilisés dans le procédé selon la présente invention on peut citer les hydrocarbures aliphatiques ou cycliques tels que le pentane, l'hexane, le cyclohexane ou l'heptane, les hydrocarbures aromatiques tels que le benzène, le toluène ou les xylènes, les solvants chlorés tels que le dichlorométhane ou le chlorobenzène, ou encore l'acétonitrile, le diéthyléther, le tétrahydrofurane (THF). Le solvant organique sera de préférence un solvant aliphatique saturé ou insaturé ou hydrocarbure aromatique.

Le solvant peut également être un liquide ionique, en mélange ou non avec un solvant organique.

La réaction d'oligomérisation sélective du butène-1 peut être conduite en système fermé, en système semi-ouvert ou en continu, avec un ou plusieurs étages de réaction. Une vigoureuse agitation doit assurer un bon contact entre le ou les réactifs et la composition catalytique.

La température de réaction peut être de -40 à +250°C, de préférence de 0°C à +150°C.

La chaleur engendrée par la réaction peut être éliminée par tous les moyens connus de l'homme du métier.

La réaction s'effectue de préférence en phase liquide, la pression est ajustée pour maintenir le système en phase liquide. Généralement, la pression peut varier de la pression atmosphérique à 10 MPa, de préférence la pression est comprise entre 5 et 8 MPa.

L'effluent de l'oligomérisation sélective comporte ainsi les oligomères issus du butène-1, notamment des octènes majoritairement linéaires, le butène-2 et optionnellement les butanes, si présents. Cet effluent est ensuite soumis à une étape de séparation, par exemple à une distillation, afin de séparer les oligomères formés du butène-2 (et des butanes si présents).

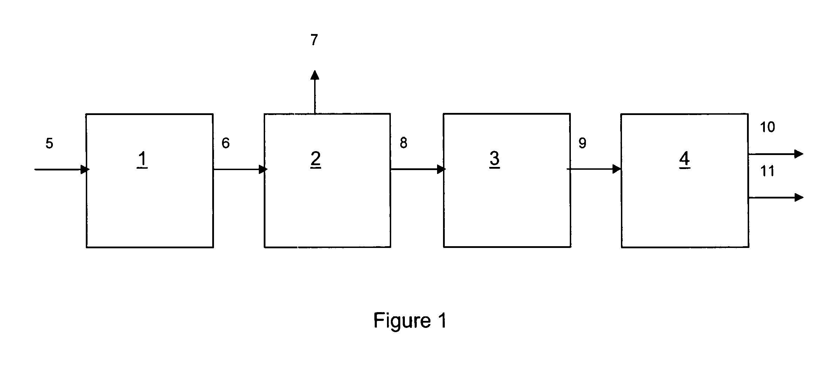

La figure 1 présente schématiquement un mode de réalisation avantageux selon l'invention. On ne reprendra pas les conditions opératoires décrites précédemment. La figure 1 décrit un procédé de séparation du butène-2 d'une charge C4 comprenant en plus du butène-2 et du butène-1, de l'isobutène, du butadiène, du n-butane, de l'isobutane et des traces d'hydrocarbures acétyléniques, ledit procédé comprend les étapes successives suivantes:
- une étape optionnelle d'hydrogénation sélective,
- une étape optionnelle de séparation de l'isobutène,
- une étape d'oligomérisation sélective du butène-1,
- puis une étape de séparation du butène-2.

La charge C4 (5) qui contient du butène-1, du butène-2, de l'isobutène, du butadiène, du n-butane, de l'isobutane et des traces d'hydrocarbures acétyléniques est soumise à une étape optionnelle d'hydrogénation sélective (1) du butadiène-1,3 avec isomérisation du butène-1 en butène-2 à l'équilibre thermodynamique et hydrogénation des hydrocarbures acétyléniques. Cette étape permet ainsi d'éliminer le butadiène-1,3 et les traces d'hydrocarbures acétyléniques. L'effluent (6) contenant du butène-1, du butène-2, de l'isobutène, du n-butane et de l'isobutane est ensuite envoyé dans une étape optionnelle (2) de séparation de l'isobutène qui peut être effectuée par éthérification, par oligomérisation ou par polymérisation sélective de l'isobutène permettant d'obtenir un flux (7) contenant du MTBE, du diisobutène ou du PIB, et un effluent (8) contenant du butène-1, du butène-2, du n-butane et de l'isobutane. Cet effluent (8) est par la suite envoyé dans une étape d'oligomérisation sélective du butène-1 (3) permettant d'obtenir un effluent (9) contenant des oligomères (principalement des octènes) majoritairement linéaires en mélange avec le butène-2, le n-butane et l'isobutane. Une étape de séparation (4), par exemple par distillation, permet ensuite de séparer les oligomères (10) d'une fraction (11) contenant le butène-2 non réagit en mélange avec le n-butane et l'isobutane.

### Exemples

### Préparation du précurseur du catalyseur :

La composition catalytique utilisée est préparée de la façon suivante. Le ligand bis(imino)pyridine (1.58 g, 1.05 éq.) et le précurseur dichlorure de fer(II) tétrahydraté (0.88 g, 1 éq.) sont ajoutés dans un Schlenk sous argon contenant un barreau magnétique. 150 mL de THF sont alors ajoutés et la solution est agitée à température ambiante pendant 16h. Le solide formé est isolé par filtration puis lavé à l'éther et au pentane (2.17 g, 91 %).

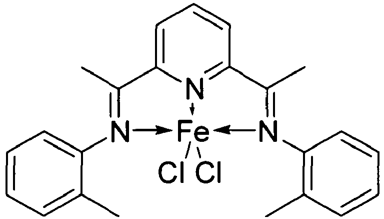

### Tests catalytiques

Les tests catalytiques (exemples 1 à 2, tableau 3) ont été effectués de la manière suivante: Le réacteur de 250 mL est séché sous vide à 80°C pendant 3h puis placé sous argon. La charge est injectée dans le réacteur puis refroidie à 0°C. Le complexe de fer (2.10⁻⁵ mol) dans 3 mL de toluène est introduit ainsi que l'activateur (MAO, Al/Fe = 105, 10 % pds dans le toluène). L'agitation est démarrée et la consigne de température fixée à 40°C. Après le temps de réaction souhaité, la neutralisation est réalisée avec H₂O. Les conversions sont suivies par analyse GC des phases gaz et liquides.

Dans l'exemple 1 (non conforme à l'invention) la charge est du butène-1. Dans i'exempie 2 (conforme à l'invention), la charge est un mélange de butène-1 et de butène -2 dont le ratio butène-1/butène-2 est de 0.85.

**Tableau 3 : Tests catalytiques**

| N° | charge | Durée (min) | Conversion du butène-2 | Dimères (% pds/total des produits) | Octènes linéaires (%pds de n-octènes/total dimères) |
|---|---|---|---|---|---|
| 1 | butène-1 | 165 | 0 | 70 | 67 |
| 2 | butène-1 + butène-2 | 360 | <5 | 73 | 65 |

L'exemple 2 montrent que la réalisation du procédé selon l'invention permet de séparer le butène-2 d'une coupe C4 contenant du butène-1 et du butène-2 par oligomérisation sélective du butène-1 en oligomères avec une sélectivité en dimères de 73 % pds et une linéarité des octènes formés de 65 % pds/total. La conversion du butène-2 reste extrêmement faible. La comparaison des exemples 1 et 2 montre que la présence du butène-2 n'influence peu la sélectivité et la linéarité.

## Revendications

1. Procédé de séparation du butène-2 d'une charge C4 comprenant du butène-1 et du butène-2 **caractérisé par** les étapes successives suivantes:
- une étape d'oligomérisation sélective du butène-1 par une composition catalytique à base de fer
- une étape de séparation des oligomères formés du butène-2.

2. Procédé selon la revendication précédente dans lequel la composition catalytique à base de fer comprend un précurseur de fer et au moins un ligand de formule décrite ci-dessous, complexé ou non au précurseur de fer, ledit précurseur de fer pouvant être hydraté ou non et a pour formule générale FeXₙ, X étant un groupement anionique choisi parmi un halogénure, un groupement hydrocarboné, un carboxylate, un oxyde, un amide, un alkoxyde, un hydroxyde ou un anion non-coordinant, ledit ligand ayant pour formule générale : 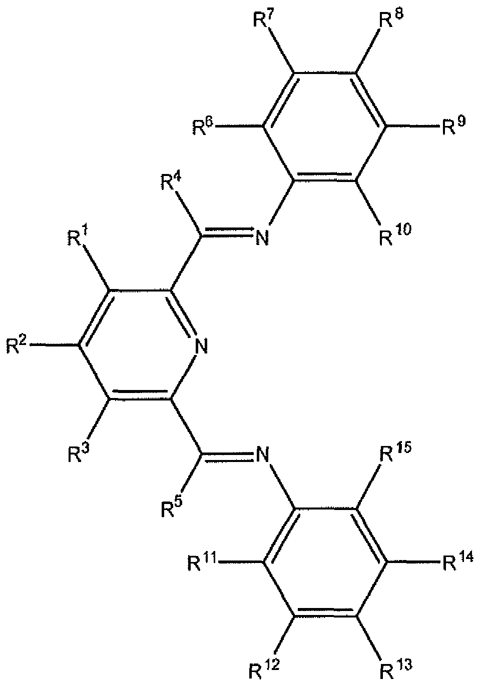 où R¹, R², R³, R⁴, R⁵, R⁶, R⁷, R⁸, R⁹, R¹⁰, R¹¹, R¹², R¹³, R¹⁴ et R¹⁵ peuvent être identiques ou différents, et sont choisis parmi l'atome d'hydrogène, les groupements alkyles linéaires ou ramifiés, cycliques ou non, saturés ou insaturés, les groupements aryles, aralkyles ou alkaryles comportant 1 à 12 atomes de carbone, les groupements contenant des hétéro-éléments, hétérocycliques ou non, aromatiques ou non, halogénures ou non, supportés ou non.

3. Procédé selon l'une des revendications précédentes dans lequel la composition catalytique comprend un activateur choisi parmi les dérivés d'aluminium et les dérivés du bore ou du zinc ou un mélange de ces dérivés.

4. Procédé selon la revendication précédente dans lequel l'activateur est choisi parmi les alkylaluminiums, les halogénures d'alkylaluminiums, et les aluminoxanes, les trialkylboranes, les tris(aryl)boranes, les (aryl)borates associés à un cation triphénylcarbénium ou à un cation ammonium trisubstitué, les dialkylzinc.

5. Procédé selon les revendications 3 ou 4 dans lequel l'activateur est un aluminoxane choisi parmi le méthylaluminoxane (MAO) et/ou l'éthylaluminoxane (EAO) et/ou parmi les aluminoxanes modifiés tels que le méthylaluminoxane modifié (MMAO).

6. Procédé selon l'une des revendications précédentes dans lequel l'étape d'oligomérisation sélective est réalisée en présence d'un solvant organique choisi parmi les hydrocarbures aliphatiques ou cycliques, les hydrocarbures aromatiques, les solvants chlorés, l'acétonitrile, le diéthyléther et/ou le tétrahydrofurane, et/ou un solvant liquide ionique.

7. Procédé selon l'une des revendications précédentes dans lequel l'étape d'oligomérisation sélective est réalisée à une température entre -40 à +250°C et à une pression variant de la pression atmosphérique à 10 MPa.

8. Procédé selon l'une des revendications précédentes dans lequel la charge contient également de l'isobutène et qu'on procède à une étape de séparation de l'isobutène avant l'étape d'oligomérisation.

9. Procédé selon la revendication précédente dans lequel la séparation de l'isobutène est effectuée par au moins une des étapes suivantes :
- éthérification de l'isobutène en un alkyl-tertiobutyléther en présence d'un catalyseur d'étherification avec un alcool dont la chaîne hydrocarbonée peut comporter de 1 à 10 atomes de carbone,
- dimérisation de l'isobutène en présence d'un catalyseur acide,
- polymérisation de l'isobutène en présence d'un catalyseur acide de Lewis.

10. Procédé selon l'une des revendications précédentes dans lequel la charge contient également du butadiène-1,3 et éventuellement des traces d'hydrocarbures acétyléniques et qu'on procède à une étape de séparation du butadiène et des éventuelles traces d'hydrocarbures acétyléniques par hydrogénation sélective avant l'étape de séparation de l'isobutène et/ou l'étape d'oligomérisation sélective.

11. Procédé selon la revendication précédente dans lequel l'hydrogénation sélective du butadiène-1,3 et des traces d'hydrocarbures acétyléniques est effectuée par un catalyseur comprenant au moins un métal choisi dans le groupe formé par le nickel, le palladium et le platine, déposé sur un support, à une température de 20-200°C, une pression de 1-5 MPa, une vitesse spatiale de 0,5-10 h⁻¹ et avec un rapport molaire H₂ / butadiène de 0,5 à 5.

12. Procédé selon les revendications précédentes dans lequel la charge provient d'une unité de craquage catalytique, d'une unité de vapocraquage et/ou d'une unité de déshydratation des alcools.

## Patentansprüche

1. Verfahren zum Abtrennen von Buten-2 aus einer C4-Beschickung, umfassend Buten-1 und Buten-2, das durch die folgenden Schritte gekennzeichnet ist:
- einen Schritt zur selektiven Oligomerisation von Buten-1 durch eine katalytische Zusammensetzung auf der Basis von Eisen,
- einen Schritt zum Abtrennen der aus dem Buten-2 gebildeten Oligomere.

2. Verfahren nach dem vorhergehenden Anspruch, wobei die katalytische Zusammensetzung auf der Basis von Eisen einen Eisenvorläufer und mindestens einen Liganden mit der unten beschriebenen Formel, mit dem Eisenvorläufer komplexiert oder nicht, umfasst, wobei der Eisenvorläufer hydratisiert sein kann oder nicht und die allgemeine Formel FeXₙ aufweist, wobei X für eine anionische Gruppe steht, ausgewählt aus einem Halogenid, einer Kohlenwasserstoffgruppe, einem Carboxylat, einem Oxid, einem Amid, einem Alkoxid, einem Hydroxid oder einem nicht oder schwach koordinierenden Anion, wobei der Ligand folgende allgemeine Formel aufweist: 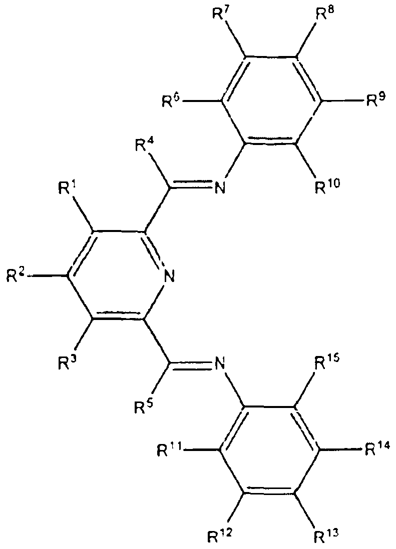 worin R¹ R², R³, R⁴, R⁵, R⁶, R⁷, R⁸, R⁹, R¹⁰, R¹¹, R¹², R¹³, R¹⁴ und R¹⁵ gleich oder verschieden sein können und ausgewählt sind aus einem Wasserstoffatom, linearen oder verzweigten, cyclischen oder nicht cyclischen, gesättigten oder ungesättigten Alkylgruppen, Aryl-, Aralkyl- oder Alkarylgruppen, umfassend 1 bis 12 Kohlenstoffatome, wobei die Gruppen heterocyclische oder nicht heterocyclische, aromatische oder nicht aromatische, halogenierte oder nicht halogenierte, geträgerte oder nicht geträgerte Heteroelemente enthalten.

3. Verfahren nach einem der vorhergehenden Ansprüche, wobei die katalytische Zusammensetzung einen Aktivator umfasst, ausgewählt aus Aluminiumderivaten und Bor- oder Zinkderivaten oder einem Gemisch aus diesen Derivaten.

4. Verfahren nach dem vorhergehenden Anspruch, wobei der Aktivator ausgevrähft ist aus Alkylaluminiumverbindungen, Alkylaluminiumhalogeniden und Aluminoxanen, Trialkylboranen, Tris(aryl)boranen, mit einem Triphenylcarbeniumkation oder mit einem trisubstituierten Ammoniumkation assoziierten (Aryl)boraten, Dialkylzinkverbindungen.

5. Verfahren nach den Ansprüchen 3 oder 4, wobei der Aktivator ein Aluminoxan ist, ausgewählt aus Methylaluminoxan (MAO) und/oder Ethylaluminoxan (EAO) und/oder aus modifizierten Aluminoxanen, wie etwa modifiziertem Methylaluminoxan (MMAO).

6. Verfahren nach einem der vorhergehenden Ansprüche, wobei der Schritt zur selektiven Oligomerisation in Gegenwart eines organischen Lösemittels ausgeführt wird, ausgewählt aus aliphatischen oder cyclischen Kohlenwasserstoffen aromatischen Kohlenwasserstoffen, chlorierten Lösemitteln, Acetonitril, Diethylether und/oder Tetrahydrofuran und/oder einem flüssigen ionischen Lösemittel.

7. Verfahren nach einem der vorhergehenden Ansprüche, wobei der Schritt zur selektiven Oligomerisation bei einer Temperatur zwischen -40 bis +250 °C und bei einem Druck, der im Bereich zwischen dem Atmosphärendruck und 10 MPa variiert, ausgeführt wird.

8. Verfahren nach einem der vorhergehenden Ansprüche, wobei die Beschickung auch Isobuten enthält und vor dem Schritt zur Oligomerisation ein Schritt zur Abtrennung des Isobutens durchgeführt wird.

9. Verfahren nach einem der vorhergehenden Ansprüche, wobei die Abtrennung des Isobutens durch mindestens einen der folgenden Schritte erfolgt:
- Veretherung des Isobutens in einen Alkyl-tert-Butylether in Gegenwart eines Veretherungskatalysators mit einem Alkohol, dessen Kohlenwasserstoffkette 1 bis 10 Kohlenstoffatome umfassen kann,
- Dimerisation des Isobutens in Gegenwart eines sauren Katalysators,
- Polymerisation des Isobutens in Gegenwart eines Lewis-Säure-Katalysators.

10. Verfahren nach einem der vorhergehenden Ansprüche, wobei die Beschickung auch Butadien-1,3 und gegebenenfalls Spuren von acetylenischen Kohlenwasserstoffen enthält, und ein Schritt zur Abtrennung von Butadien und gegebenenfalls vorhandenen Spuren von acetylenischen Kohlenwasserstoffen durch selektive Hydrierung vor dem Schritt zur Abtrennung des Isobutens und/oder dem Schritt zur selektiven Oligomerisation durchgeführt wird.

11. Verfahren nach einem dem vorhergehenden Anspruch, wobei die selektive Hydrierung von Butadien-1,3 und von Spuren von acetylenischen Kohlenwasserstoffen mit einem Katalysator umfassend mindestens ein Metall, ausgewählt aus der Gruppe gebildet aus Nickel, Palladium und Platin, abgelagert auf einem Träger, bei einer Temperatur von 20 bis 200 °C, einem Druck von 1 bis 5 MPa, einer Raumgeschwindigkeit von 0,5 bis 10 h⁻¹ und mit einem H₂/Butadien-Verhältnis (in Mol) von 0,5 bis 5 durchgeführt wird.

12. Verfahren nach einem der vorhergehenden Ansprüche, wobei die Beschickung aus einer Einheit zum katalytischen Cracken, einer Einheit zum Dampferacken und/oder einer Einheit zur Dehydratisierung von Alkoholen stammt.

## Claims

1. Method of separating butene-2 from a C4 feedstock comprising butene-1 and butene-2, **characterized by** the following successive steps:
- a step of selective oligomerization of butene-1 by an iron-based catalytic composition
- a step of separation of the oligomers formed from the butene-2.

2. Method according to the previous claim in which the iron-based catalytic composition comprises an iron precursor and at least one ligand of the formula described below, complexed or not complexed with the iron precursor, and said iron precursor can be hydrated or not and has the general formula FeXₙ, X being an anionic group chosen from a halide, a hydrocarbon group, a carboxylate, an oxide, an amide, an alkoxide, a hydroxide or a non-coordinating anion, said ligand having the general formula: 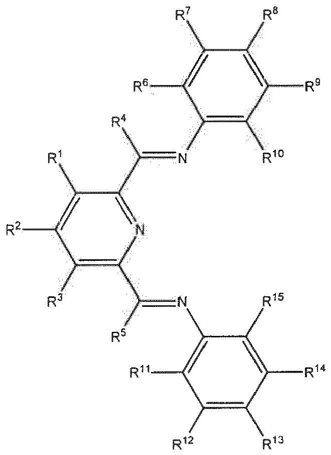 where R¹, R², R³, R⁴, R⁵, R⁶, R⁷, R⁸, R⁹, R¹⁰, R¹¹, R¹², R¹³, R¹⁴ and R¹⁵, which may be identical or different, are chosen from the hydrogen atom, linear or branched, cyclic or non-cyclic, saturated or unsaturated alkyl groups, aryl, aralkyl or alkaryl groups comprising 1 to 12 carbon atoms, groups containing hetero-elements, heterocyclic or not, aromatic or not, halides or not, supported or not.

3. Method according to one of the previous claims in which the catalytic composition comprises an activator chosen from aluminium derivatives and boron or zinc derivatives or a mixture of said derivatives.

4. Method according to the previous claim in which the activator is chosen from the alkylaluminiums, alkylaluminium halides, and aluminoxanes, trialkylboranes, tris(aryl)boranes, (aryl)borates associated with a triphenylcarbenium cation or with a trisubstituted ammonium cation, dialkylzincs.

5. Method according to claims 3 or 4 in which the activator is an aluminoxane chosen from methylaluminoxane (MAO) and/or ethylaluminoxane (EAO) and/or from modified aluminoxanes such as modified ethylaluminoxane (MAO).

6. Method according to one of the previous claims in which the step of selective oligomerization is carried out in the presence of an organic solvent chosen from aliphatic or cyclic hydrocarbons, aromatic hydrocarbons, chlorinated solvents, acetonitrile, diethyl ether and/or tetrahydrofuran, and/or an ionic liquid solvent.

7. Method according to one of the previous claims in which the step of selective oligomerization is carried out at a temperature between -40 and +250°C and at a pressure varying from atmospheric pressure to 10 MPa.

8. Method according to one of the previous claims in which the feedstock also contains isobutene and a step of separation of the isobutene is carried out before the oligomerization step.

9. Method according to the previous claim in which the separation of isobutene is carried out by at least one of the following steps:
- etherification of isobutene to an alkyl tert-butyl ether in the presence of an etherification catalyst with an alcohol the hydrocarbon chain of which can comprise from 1 to 10 carbon atoms,
- dimerization of the isobutene in the presence of an acid catalyst,
- polymerization of the isobutene in the presence of a Lewis acid catalyst.

10. Method according to one of the previous claims in which the feedstock also contains 1,3-butadiene and optionally traces of acetylene hydrocarbons and a step of separation of butadiene and any traces of acetylene hydrocarbons by selective hydrogenation is carried out before the step of separation of isobutene and/or the step of selective oligomerization.

11. Method according to the previous claim in which the selective hydrogenation of 1,3-butadiene and traces of acetylene hydrocarbons is carried out with a catalyst comprising at least one metal chosen from the group formed by nickel, palladium and platinum, deposited on a support, at a temperature of 20-200°C, a pressure of 1-5 MPa, a space velocity of 0.5-10 h⁻¹ and with an H₂ / butadiene molar ratio of 0.5 to 5.

12. Method according to the previous claims in which the feedstock originates from a catalytic cracking unit, from a steam cracking unit and/or from an alcohol dehydration unit.
